(19) Europäisches Patentamt
European Patent Office
Office européen des brevets

(11) EP 4 718 467 A1

(12) EUROPEAN PATENT APPLICATION

(43) Date of publication:
01.04.2026 Bulletin 2026/14

(21) Application number: 24465578.3

(22) Date of filing: 27.09.2024

(51) International Patent Classification (IPC):
G16H 30/40 (2018.01)     G16H 50/20 (2018.01)

(52) Cooperative Patent Classification (CPC):
G16H 50/20; G16H 30/40

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Siemens Healthineers AG
91301 Forchheim (DE)

(72) Inventors:
• GULSUN, Mehmet Akif
  Princeton, NJ 08540 (US)
• SHARMA, Puneet
  Princeton Junction, NJ 08550 (US)
• SERBAN, Alexandru Constantin
  900520 Constanta (RO)
• COMANICIU, Dorin
  Princeton, NJ 08540 (US)

(74) Representative: HKW Intellectual Property PartG
mbB
Theresienhöhe 12
80339 München (DE)

(54) **AI SYSTEM FOR AUTOMATIC INTERACTION WITH CLINICAL INFORMATION SYSTEMS AND MEDICAL APPLICATIONS**

(57)     Systems and methods for automatic interaction between a user and one or more clinical information systems and one or more medical applications are provided. First text-based instructions are received from a user. 1) Second text-based instructions for retrieving clinical information and 2) third text-based instructions for performing a medical analysis task are generated by an interface AI agent based on the first text-based instructions. One or more actions for retrieving the clinical information from one or more clinical information systems are determined by a data AI agent based on the second text-based instructions. One or more actions for executing on one or more medical applications to perform the medical analysis task are determined by a task AI agent based on the third text-based instructions. The clinical information and results of the medical analysis task are output.

FIG. 2

**Description**

TECHNICAL FIELD

The present invention relates generally to AI/ML (artificial intelligence/machine learning) systems, and in particular to an AI/ML system for automatic interaction with clinical information systems and medical applications.

BACKGROUND

Medical imaging applications play an important role in the hospital setting by facilitating the acquisition, processing, and analysis of medical images, thereby enabling the diagnosis, monitoring, and treatment of various medical conditions. Such medical imaging applications are software applications typically programmed to perform specific, pre-defined tasks. However, navigating between various medical imaging applications can be cumbersome and time-consuming, and may result in suboptimal assessment of the medical images and unfulfillment of user needs. This is because users must typically manually browse and load data for input into such medical imaging applications and medical imaging applications typically do not adapt to contextual information around the patient and clinicians. Further, the format, structure, and availability of patient data varies across clinical sites and each clinical site may have its own configuration for accessing patient database.

BRIEF SUMMARY OF THE INVENTION

In accordance with one or more embodiments, systems and methods for automatic interaction between a user and clinical information systems and medical applications are provided. First text-based instructions are received from a user. 1) Second text-based instructions for retrieving clinical information and 2) third text-based instructions for performing a medical analysis task are generated by an interface AI agent based on the first text-based instructions. One or more actions for retrieving the clinical information from one or more clinical information systems are determined by a data AI agent based on the second text-based instructions. One or more actions for executing on one or more medical applications to perform the medical analysis task are determined by a task AI agent based on the third text-based instructions. The clinical information and results of the medical analysis task are output.

In one embodiment, additional text-based instructions for retrieving additional clinical information are determined by the task AI agent based on the third text-based instructions. One or more actions for retrieving the additional clinical information are determined by the data AI agent based on the additional text-based instructions. The one or more actions for executing on the one or more medical applications are determined by the task AI agent further based on the additional clinical information.

In one embodiment, text-based follow-up instructions requesting additional information from the user are generated by the interface AI agent based on the first text-based instructions. 1) The second text-based instructions and 2) the third text-based instructions are generated by the interface AI agent further based on a response to the text-based follow-up instructions received from the user.

In one embodiment, the one or more actions for retrieving the clinical information comprises at least one of: searching, classifying, parsing, or interpreting clinical data stored in the one or more clinical information systems.

In one embodiment, the one or more actions for executing on the one or more medical applications comprise at least one of: one or more medical image analysis tasks performed on one or more medical images, functions to derive findings from the one or more medical images, functions to apply geometric transformations on the one or more medical images, functions to retrieve geometric information from the one or more medical images, or outputting text-based instructions to a machine learning based model.

In one embodiment, at least one of the one or more actions for retrieving the clinical information or the one or more actions for executing on the one or more medical applications comprise one or more APIs (application programming interfaces).

In one embodiment, spoken instructions are received from the user. The spoken instructions are converted to the first text-based instructions.

In one embodiment, the interface AI agent, the data AI agent, and the task AI agent each comprise a machine learning based text encoder network and a policy module. The machine learning based text encoder network comprises a language model.

In one embodiment, the first text-based instructions are at least one of predefined based on a specific medical application or automatically generated based on contextual information of the user or patient.

These and other advantages of the invention will be apparent to those of ordinary skill in the art by reference to the following detailed description and the accompanying drawings.

BRIEF DESCRIPTION OF THE DRAWINGS

Figure 1 shows a method for automatically interacting with clinical information systems and medical applications, in accordance with one or more embodiments;

Figure 2 shows a workflow for automatically interacting with clinical information systems and medical applications, in accordance with one or more embodiments;

Figure 3 shows an exemplary artificial neural network that may be used to implement one or more embodiments;

Figure 4 shows a convolutional neural network that may be used to implement one or more embodiments;

Figure 5 shows a schematic structure of a recurrent machine learning model that may be used to implement one or more embodiments; and

Figure 6 shows a high-level block diagram of a computer that may be used to implement one or more embodiments.

DETAILED DESCRIPTION

The present invention generally relates to AI systems and methods for automatic interaction with clinical information systems and medical applications. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

Embodiments described herein provide for an AI system comprising a data AI agent, a task AI agent, and an interface AI agent for automatic interaction between a user and clinical information systems and medical applications. Each AI agent comprises a multi-modal image-text foundational model and a policy module. The foundational model understands and decomposes natural language text-based instructions from users or other AI agents into features. The policy module maps the features to one or more actions for generating a response to the text-based instructions from the user. Advantageously, the data AI agent, the task AI agent, and the interface AI agent interact with each other to provide seamless, on-demand, context specific, automatic interaction between users and clinical information systems and medical applications.

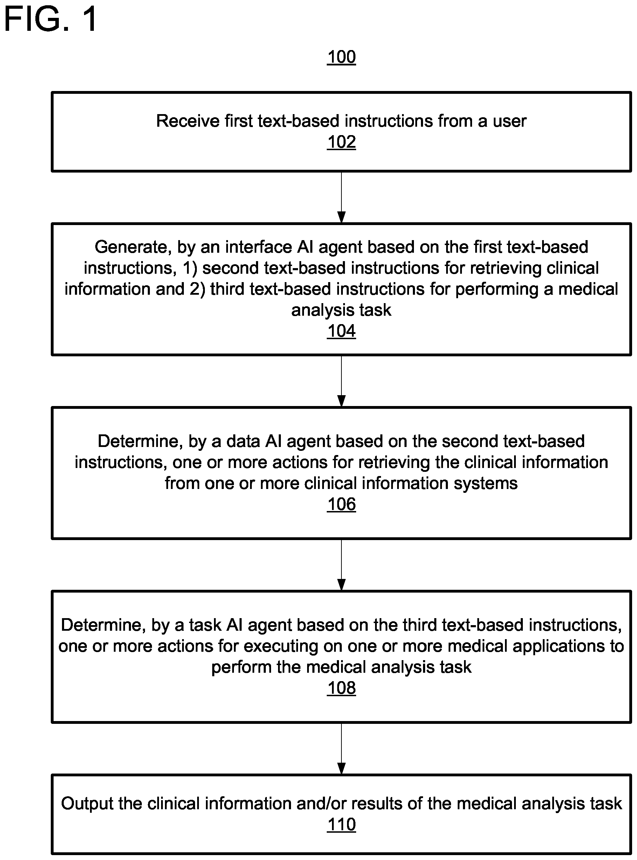

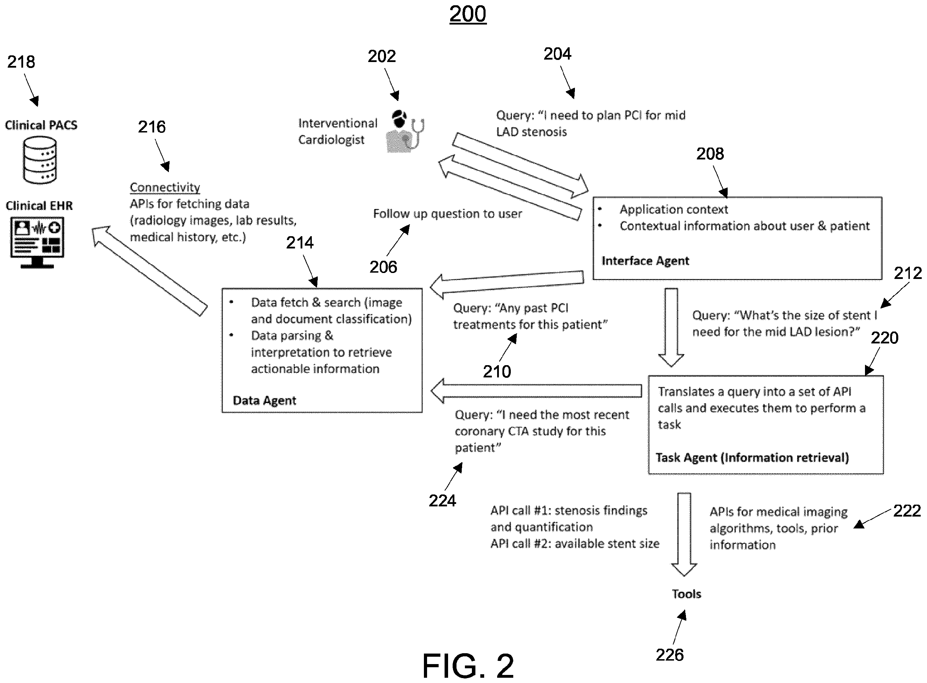

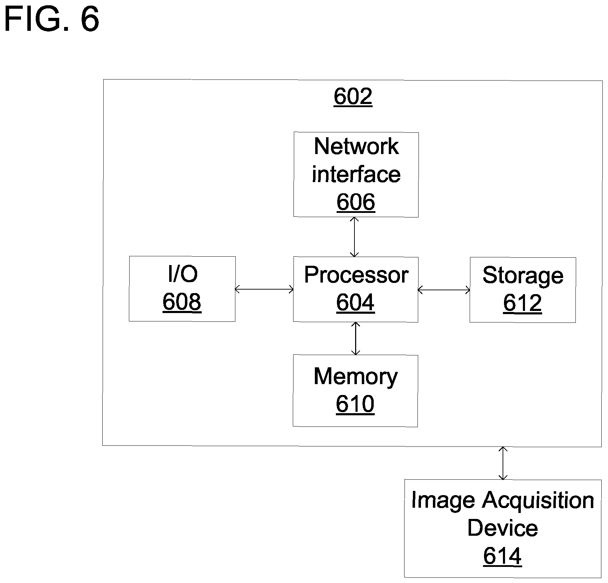

Figure 1 shows a method 100 for automatically interacting with clinical information systems and medical applications, in accordance with one or more embodiments. The steps and sub-steps of method 100 may be performed by one or more suitable computing devices, such as, e.g., computer 602 of Figure 6. Figure 2 shows a workflow 200 for automatically interacting with clinical information systems and medical applications, in accordance with one or more embodiments. Figure 1 and Figure 2 will be described together.

At step 102 of Figure 1, first text-based instructions are received from a user. The user may be a clinician or any other user. In one example, as shown in workflow 200 of Figure 2, the first text-based instructions are query 204 received by interface AI agent 208 from interventional cardiologist 202..

The first text-based instructions may comprise natural language text-based commands or queries from the user. For example, in workflow 200 of Figure 2, query 204 comprises the query "I need to plan PCI for mid LAD stenosis", where PCI refers to percutaneous coronary intervention and LAD refers to the left anterior descending coronary artery. The first text-based instructions may be predefined based on the specific medical application or automatically generated based on contextual information of the user and/or patient. In one embodiment, spoken instructions are first received from the user (e.g., via a microphone) and the spoken instructions are converted to the first text-based instructions using, e.g., any well-known speech-to-text translator.

In one embodiment, optionally, one or more medical images are also received. The one or more medical images may depict an anatomical object, such as, e.g., organs, bones, vessels, tumors or other abnormalities, or any other anatomical object of interest of a patient. The one or more medical images may be associated with the text-based instructions. For example, the text-based instructions may be instructions for modifying, extracting information from, or otherwise analyzing the one or more medical images. The one or more medical images may be of any suitable modality, such as, e.g., MRI (magnetic resonance imaging), PET (positron emission tomography), SPECT (single photon emission computed tomography), CT (computed tomography), US (ultrasound), x-ray, or any other medical imaging modality or combinations of medical imaging modalities. The one or more medical images may be 2D (two dimensional) images and/or 3D (three dimensional) volumes, and may comprise a single image or a plurality of images.

The first text-based instructions and/or one or more medical images may be received, for example, by directly receiving the first text-based instructions from a user via an input/output (I/O) device (e.g., I/O 608 of Figure 6), by directly receiving the one or more medical images from an medical image acquisition device (e.g., image acquisition device 614 of Figure 6) as the images are acquired, by loading the first text-based instructions and/or one or more medical images from a storage or memory of a computer system (e.g., storage 612 or memory 610 of computer 602 of Figure 6), or by receiving the first text-based instructions and/or one or more medical images from a remote computer system (e.g., computer 602 of Figure 6). Such a computer system or remote computer system may comprise one or more clinical information systems, such as, e.g. , an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 104 of Figure 1, 1) second text-based instructions for retrieving clinical information and 2) third text-based instructions for performing a medical analysis task are generated by an interface AI agent based on the first text-based instructions. In one example, as shown in workflow 200 of Figure 2, the interface AI agent is interface AI agent 208 for determining query 210 for retrieving clinical information as the second text-based instructions and query 212 for performing a medical analysis task as the third text-based instructions.

The second and third text-based instructions may comprise natural language text-based commands or queries. For example, in workflow 200 of Figure 2, query 210 comprises "Any past PCI treatments for this patient?" and query 212 comprises "What's the size of stent I need for the mid LAD lesion?"

The interface AI agent translates the first text-based instructions received from the user into second and third text-based instructions for input to the data AI agent and task AI agent respectively. The interface AI agent generates the second and third text-based instructions based on application context and contextual information about the user and the patient. The interface AI agent may be implemented according to any suitable machine learning based architecture. In one embodiment, the interface AI agent comprises a foundational model (comprising a machine learning based text encoder network and optionally a machine learning based image encoder) and a policy module. The machine learning based text encoder network receives as input the first text-based instructions and generates as output the text features. The machine learning based image encoder network receives as input the one or more medical images and generates as output the image features. The policy module maps the text and/or image features to the second text-based instructions and the third text-based instructions.

In one embodiment, the interface AI agent can generate text-based follow-up instructions to the user based on the first text-based instructions, for example, requesting additional information. The text-based follow-up instructions may be generated by the interface AI agent based on the first text-based instructions, the application context, contextual information about the user and patient, or any text-based instructions received from the data AI agent and task AI agent. In one example, as shown in workflow 200 of Figure 2, interface AI agent 208 generates follow up question 206 to interventional cardiologist 202.

At step 106 of Figure 1, one or more actions for retrieving the clinical information from one or more clinical information systems are determined by a data AI agent based on the second text-based instructions. In one example, as shown in workflow 200 of Figure 2, data AI agent 214 generates actions 216 for retrieving clinical information from clinical information systems 218 based on query 210.

The one or more actions for retrieving the clinical information may include any suitable actions for retrieving the clinical information from the one or more clinical information systems. For example, the one or more actions for retrieving the clinical information may comprise searching, classifying, parsing, interpreting clinical data stored in the one or more clinical information systems. The clinical data may comprise, for example, medical history, diagnoses, medical images, laboratory results, reports, or any other data relating to patient. In one embodiment, the one or more actions for retrieving the clinical information comprise APIs (application programming interfaces) for communicating with one or more medical applications.

The data AI agent maps the second text-based instructions into the one or more actions for retrieving the clinical information. The data AI agent has physical connections to the one or more clinical information systems and can retrieve the clinical information. Based on the second text-based instructions, the data AI agent is trained to determine the one or more actions for retrieving the clinical information. The data AI agent parses the retrieved information to extract, generate, and present the clinical information as a response to the second text-based instructions.

The data AI agent may be implemented according to any suitable machine learning based architecture. In one embodiment, similar to the interface AI agent, the data AI agent comprises a foundational model (comprising a machine learning based text encoder network) and a policy module. The machine learning based text encoder network receives as input the second text-based instructions and generates as output the text features. The policy module maps the text features to the one or more actions for retrieving the clinical information.

At step 108 of Figure 1, one or more actions for executing on one or more medical applications to perform the medical analysis task are determined by a task AI agent based on the third text-based instructions. In one example, as shown in workflow 200 of Figure 2, task AI agent 220 generates actions 222 for performing on medical applications 226 based on query 212. Actions 222 comprises an action for stenosis findings and quantification and an action for determining the available stent size.

The one or more actions for executing on the one or more medical applications may include any suitable actions for executing on one or more medical applications to perform the medical analysis task. For example, the one or more actions for executing on the one or more medical applications may comprise at least one of: 1) one or more medical image analysis tasks performed on the one or more medical images (e.g., medical image detection, classification, and segmentation using machine learning based models), 2) functions to derive measurements or other findings from the one or more medical images (e.g., stenosis measurement from coronary segmentations, CAD-RADS (coronary artery disease - reporting and data systems) findings from stenosis measurements in detected segmentations), 3) functions to apply geometric transformations on the one or more medical images (e.g., rotate an image by a particular angle), 4) functions to retrieve geometric information from the one or more medical images (e.g., whether the specific mesh is blocked in a given camera angle), or 5) outputting text-based instructions to another AI agent, machine learning based network, or medical application. In one embodiment, the one or more actions comprise one or more APIs for communicating with the one or more medical applications. The one or more medical applications may comprise any medical related application, such as, e.g., software applications, machine learning based models, etc.

In one embodiment, the one or more actions for executing on the one or more medical applications comprise one or more text-based instructions output to the data AI agent for retrieving additional clinical information. For example, as shown in workflow 200 of Figure 2, task AI agent 220 generates query 224 for retrieving additional clinical information. Query 224 comprises the query: "I need the most recent coronary CTA study for this patient", where CTA refers to CT angiography. Data AI agent 214 receives query 224, retrieves the additional clinical information based on query 224, and returns the additional clinical information to task AI agent 220. Task AI agent 220 then generates actions 222 further based on the additional clinical information retrieved in response to query 224.

The task AI agent maps the third text-based instructions to the one or more actions for executing on the one or more medical applications and executes the one or more actions on the one or more medical applications to perform the medical analysis task. The task AI agent may be implemented according to any suitable machine learning based architecture. In one embodiment, similar to the interface AI agent, the task AI agent comprises a foundational model (comprising a machine learning based text encoder network) and a policy module. The machine learning based text encoder network receives as input the third text-based instructions and generates as output the text features. The policy module maps the text features to the one or more actions for executing on the one or more medical applications.

At step 110 of Figure 1, the clinical information and/or results of the medical analysis task are output. For example, the clinical information and/or results of the medical analysis task can be output by displaying the clinical information and/or results of the medical analysis task on a display device of a computer system (e.g., I/O 608 of computer 602 of Figure 6), storing the clinical information and/or results of the medical analysis task on a memory or storage of a computer system (e.g., memory 610 or storage 612 of computer 602 of Figure 6), or by transmitting the clinical information and/or results of the medical analysis task to a remote computer system (e.g., computer 602 of Figure 6).

As discussed above, the interface AI agent, the data AI agent, and the task AI agent comprise a foundational model (comprising a machine learning based text encoder network and optionally a machine learning based image encoder network) and a policy module. The foundational model may be any well-known, off-the-shelf multi-modal image-text foundational model comprising a machine learning based text encoder network and optionally a machine learning based image encoder network. For example, the multi-modal image-text foundational model may be BiomedCLIP. The machine learning based text encoder network and the machine learning based image encoder network may be implemented according to any suitable machine learning based architecture. For example, the machine learning based image encoder network may be implanted as, e.g., an autoencoder, a vision transformer, a CNN (convolutional neural network), etc. In another example, the machine learning based text encoder network is implemented as a language model, such as, e.g., an LLM (large language model). However, the language model may be any other suitable language model. For example, the language model may be a small language model, which uses a relatively smaller neural network, has fewer parameters, and is trained on less training data as compared with an LLM.

The LLM may be any suitable pretrained deep learning based LLM. For example, the LLM may be based on the transformer architecture, which uses an attention mechanism to capture long-range dependencies in text. One example of a transformer-based architecture is GPT (generative pre-training transformer), which has a multilayer transformer decoder architecture that may be pretrained to optimize the next token prediction task and then fine-tuned with labelled data for various downstream tasks. Other exemplary transformer-based architectures include BLOOM (BigScience Large Open-science Open-access Multilingual Language Model) and BERT (Bidirectional Encoder Representations from Transformers).

The policy module may be implemented according to any suitable approach. In one embodiment, the policy module is implemented as a neural network for mapping the input features to the output according to a learned policy. The policy is learned during training and defines the mapping from the input to the output of the policy module.

The machine learning based text encoder network, the machine learning based image encoder network, and the policy module are trained during a prior offline or training stage. The machine learning based text encoder network, the machine learning based image encoder network, and the policy module may be jointly trained or separately trained. During training, the one or more actions are defined in text, together with thein input and output parameters and a short text description of their scope. The definitions of the one or more actions may be input to the policy module, or may be combined with training text-based instructions or training text-based instructions/image pairs and input to the policy module. The policy module is trained using training text-based instructions or training text-based instructions/image pair with their expected outcomes. In one embodiment, the expected outcomes may be simulated either following predefined workflows or by caching results from user interactions. Alternative text-based instructions may also be generated using a language model.

The policy module is trained to optimize a policy using policy optimization algorithms by adjusting policy parameters to maximize expected rewards. Parameters of the policy module are updated based on feedback from the environment using reinforcement learning. The policy optimization algorithms update the policy using optimization techniques, such as, e.g., gradient descent.

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model, such as, e.g., the interface AI agent utilized at step 104, the data AI agent utilized at step 106, and the task AI agent utilized at step 108 of Figure 1 and interface AI agent 208, data AI agent 214, and task AI agent 220 of Figure 2, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

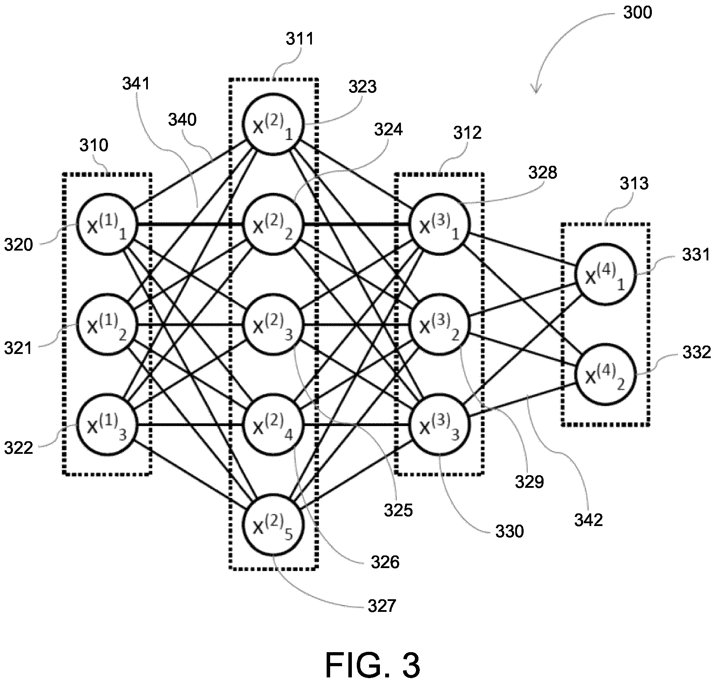

Figure 3 shows an embodiment of an artificial neural network 300 that may be used to implement one or more machine learning models described herein. Alternative terms for "artificial neural network" are "neural network", "artificial neural net" or "neural net".

The artificial neural network 300 comprises nodes 320, ..., 332 and edges 340, ..., 342, wherein each edge 340, ..., 342 is a directed connection from a first node 320, ..., 332 to a second node 320, ..., 332. In general, the first node 320, ..., 332 and the second node 320, ..., 332 are different nodes 320, ..., 332, it is also possible that the first node 320, ..., 332 and the second node 320, ..., 332 are identical. For example, in Figure 3 the edge 340 is a directed connection from the node 320 to the node 323, and the edge 342 is a directed connection from the node 330 to the node 332. An edge 340, ..., 342 from a first node 320, ..., 332 to a second node 320, ..., 332 is also denoted as "ingoing edge" for the second node 320, ..., 332 and as "outgoing edge" for the first node 320, ..., 332.

In this embodiment, the nodes 320, ..., 332 of the artificial neural network 300 can be arranged in layers 310, ..., 313, wherein the layers can comprise an intrinsic order introduced by the edges 340, ..., 342 between the nodes 320, ..., 332. In particular, edges 340, ..., 342 can exist only between neighboring layers of nodes. In the displayed embodiment, there is an input layer 310 comprising only nodes 320, ..., 322 without an incoming edge, an output layer 313 comprising only nodes 331, 332 without outgoing edges, and hidden layers 311, 312 in-between the input layer 310 and the output layer 313. In general, the number of hidden layers 311, 312 can be chosen arbitrarily. The number of nodes 320, ..., 322 within the input layer 310 usually relates to the number of input values of the neural network, and the number of nodes 331, 332 within the output layer 313 usually relates to the number of output values of the neural network.

In particular, a (real) number can be assigned as a value to every node 320, ..., 332 of the neural network 300. Here, $x^{(n)}_i$ denotes the value of the i-th node 320, ..., 332 of the n-th layer 310, ..., 313. The values of the nodes 320, ..., 322 of the input layer 310 are equivalent to the input values of the neural network 300, the values of the nodes 331, 332 of the output layer 313 are equivalent to the output value of the neural network 300. Furthermore, each edge 340, ..., 342 can comprise a weight being a real number, in particular, the weight is a real number within the interval [-1, 1] or within the interval [0, 1]. Here, $w^{(m,n)}_{i,j}$ denotes the weight of the edge between the i-th node 320, ..., 332 of the m-th layer 310, ..., 313 and the j-th node 320, ..., 332 of the n-th layer 310, ..., 313. Furthermore, the abbreviation $w^{(n)}_{i,j}$ is defined for the weight $w^{(n,n+1)}_{i,j}$.

In particular, to calculate the output values of the neural network 300, the input values are propagated through the neural network. In particular, the values of the nodes 320, ... , 332 of the (n+1)-th layer 310, ... , 313 can be calculated based on the values of the nodes 320, ... , 332 of the n-th layer 310, ... , 313 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 310 are given by the input of the neural network 300, wherein values of the first hid-den layer 311 can be calculated based on the values of the input layer 310 of the neural network, wherein values of the second hidden layer 312 can be calculated based in the values of the first hidden layer 311, etc.

In order to set the values $w^{(m,n)}_{i,j}$ for the edges, the neural network 300 has to be trained using training data. In particular, training data comprises training input data and training output data (denoted as $t_i$). For a training step, the neural network 300 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 300 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)_j} = \left(\sum_k \delta^{(n+1)_k} \cdot w^{(n+1)_{j,k}}\right) \cdot f'\left(\sum_i x^{(n)_i} \cdot w^{(n)_{i,j}}\right)$$

based on $\delta^{(n+1)}_j$, if the (n+1)-th layer is not the output layer, and

$$\delta^{(n)_j} = \left(x^{(n+1)_j} - t^{(n+1)_j}\right) \cdot f'\left(x^{(n)_i} \cdot w^{(n)_{i,j}}\right)$$

if the (n+1)-th layer is the output layer 313, wherein f' is the first derivative of the activation function, and $t^{(n+1)}_j$ is the comparison training value for the j-th node of the output layer 313.

A convolutional neural network is a neural network that uses a convolution operation instead general matrix multiplication in at least one of its layers (so-called "convolutional layer"). In particular, a convolutional layer performs a dot product of one or more convolution kernels with the convolutional layer's input data / image, wherein the entries of the one or more convolution kernel are the parameters or weights that are adapted by training. In particular, one can use the Frobenius inner product and the ReLU activation function. A convolutional neural network can comprise additional layers, e.g., pooling layers, fully connected layers, and normalization layers.

By using convolutional neural networks input images can be processed in a very efficient way, because a convolution operation based on different kernels can extract various image features, so that by adapting the weights of the convolution kernel the relevant image features can be found during training. Furthermore, based on the weight-sharing in the convolutional kernels less parameters need to be trained, which prevents overfitting in the training phase and allows to have faster training or more layers in the network, improving the performance of the network.

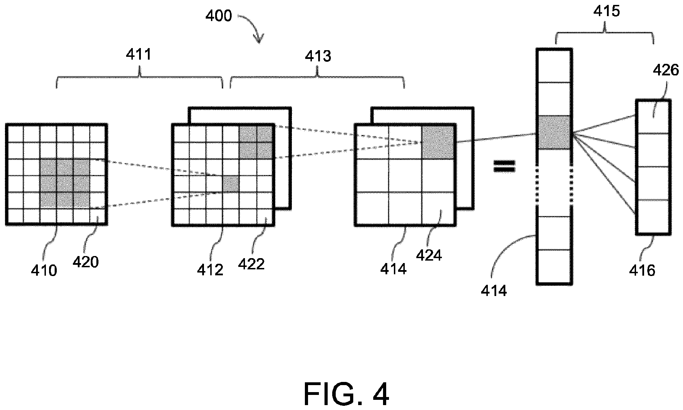

Figure 4 shows an embodiment of a convolutional neural network 400 that may be used to implement one or more machine learning models described herein. In the displayed embodiment, the convolutional neural network comprises 400 an input node layer 410, a convolutional layer 411, a pooling layer 413, a fully connected layer 414 and an output node layer 416, as well as hidden node layers 412, 414. Alternatively, the convolutional neural network 400 can comprise several convolutional layers 411, several pooling layers 413 and several fully connected layers 415, as well as other types of layers. The order of the layers can be chosen arbitrarily, usually fully connected layers 415 are used as the last layers before the output layer 416.

In particular, within a convolutional neural network 400 nodes 420, 422, 424 of a node layer 410, 412, 414 can be considered to be arranged as a d-dimensional matrix or as a d-dimensional image. In particular, in the two-dimensional case the value of the node 420, 422, 424 indexed with i and j in the n-th node layer 410, 412, 414 can be denoted as x(n)[i, j]. However, the arrangement of the nodes 420, 422, 424 of one node layer 410, 412, 414 does not have an effect on the calculations executed within the convolutional neural network 400 as such, since these are given solely by the structure and the weights of the edges.

A convolutional layer 411 is a connection layer between an anterior node layer 410 (with node values x(n-1)) and a posterior node layer 412 (with node values x(n)). In particular, a convolutional layer 411 is characterized by the structure and the weights of the incoming edges forming a convolution operation based on a certain number of kernels. In particular, the structure and the weights of the edges of the convolutional layer 411 are chosen such that the values x(n) of the nodes 422 of the posterior node layer 412 are calculated as a convolution x(n) = K * x(n-1) based on the values x(n-1) of the nodes 420 anterior node layer 410, where the convolution * is defined in the two-dimensional case as

$$x_k^{(n)}[i,j] = \left(K \ *x^{(n-1)}\right)[i,j] = \sum_{i'}\sum_{j'} K \ [i',j']\cdot x^{(n-1)}[i-i', j-j'] .$$

Here the kernel K is a d-dimensional matrix (in this embodiment, a two-dimensional matrix), which is usually small compared to the number of nodes 420, 422 (e.g., a 3x3 matrix, or a 5x5 matrix). In particular, this implies that the weights of the edges in the convolution layer 411 are not independent, but chosen such that they produce said convolution equation. In particular, for a kernel being a 3x3 matrix, there are only 9 independent weights (each entry of the kernel matrix corresponding to one independent weight), irrespectively of the number of nodes 420, 422 in the anterior node layer 410 and the posterior node layer 412.

In general, convolutional neural networks 400 use node layers 410, 412, 414 with a plurality of channels, in particular, due to the use of a plurality of kernels in convolutional layers 411. In those cases, the node layers can be considered as (d+1)-dimensional matrices (the first dimension indexing the channels). The action of a convolutional layer 411 is then a two-dimensional example defined as

$$x^{(n)_b}[i,j]= \sum_a K_{a,b}*x^{(n-1)_a}[i,j]= \sum_a\sum_{i'}\sum_{j'} K_{a,b}[i',j']\cdot x^{(n-1)_a}[i-i',j-j']$$

where $x^{(n-1)_a}$ corresponds to the a-th channel of the anterior node layer 410, $x^{(n)_b}$ corresponds to the b-th channel of the posterior node layer 412 and $K_{a,b}$ corresponds to one of the kernels. If a convolutional layer 411 acts on an anterior node layer 410 with A channels and outputs a posterior node layer 412 with B channels, there are A B independent d-dimensional kernels $K_{a,b}$.

In general, in convolutional neural networks 400 activation functions are used. In this embodiment re ReLU (acronym for "Rectified Linear Units") is used, with R(z) = max(0, z), so that the action of the convolutional layer 411 in the two-dimensional example is

$$x^{(n)_b}[i,j]= R\left(\sum_a\left(K_{a,b} * x^{(n-1)_a}\right)[i,j]\right) = R\left(\sum_a\sum_{i'}\sum_{j'} K_{a,b}[i',j']\cdot x^{(n-1)_a}[i-i',j-j']\right)$$

It is also possible to use other activation functions, e.g., ELU (acronym for "Exponential Linear Unit"), LeakyReLU, Sigmoid, Tanh or Softmax.

In the displayed embodiment, the input layer 410 comprises 36 nodes 420, arranged as a two-dimensional 6x6 matrix. The first hidden node layer 412 comprises 72 nodes 422, arranged as two two-dimensional 6x6 matrices, each of the two matrices being the result of a convolution of the values of the input layer with a 3x3 kernel within the convolutional layer 411. Equivalently, the nodes 422 of the first hidden node layer 412 can be interpreted as arranged as a three-dimensional 2x6x6 matrix, wherein the first dimension correspond to the channel dimension.

The advantage of using convolutional layers 411 is that spatially local correlation of the input data can exploited

8

by enforcing a local connectivity pattern between nodes of adjacent layers, in particular by each node being connected to only a small region of the nodes of the preceding layer.

A pooling layer 413 is a connection layer between an anterior node layer 412 (with node values x(n-1)) and a posterior node layer 414 (with node values x(n)). In particular, a pooling layer 413 can be characterized by the structure and the weights of the edges and the activation function forming a pooling operation based on a non-linear pooling function f. For example, in the two-dimensional case the values x(n) of the nodes 424 of the posterior node layer 414 can be calculated based on the values x(n-1) of the nodes 422 of the anterior node layer 412 as

$$x^{(n)_b}[i,j] = f(x^{(n-1)}[id_1, jd_2], ..., x^{(n-1)_b}[(i+1)d_1-1, (j+1)d_2-1])$$

In other words, by using a pooling layer 413 the number of nodes 422, 424 can be reduced, by re-placing a number d1 d2 of neighboring nodes 422 in the anterior node layer 412 with a single node 422 in the posterior node layer 414 being calculated as a function of the values of said number of neighboring nodes. In particular, the pooling function f can be the max-function, the average or the L2-Norm. In particular, for a pooling layer 413 the weights of the incoming edges are fixed and are not modified by training.

The advantage of using a pooling layer 413 is that the number of nodes 422, 424 and the number of parameters is reduced. This leads to the amount of computation in the network being reduced and to a control of overfitting.

In the displayed embodiment, the pooling layer 413 is a max-pooling layer, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

In general, the last layers of a convolutional neural network 400 are fully connected layers 415. A fully connected layer 415 is a connection layer between an anterior node layer 414 and a posterior node layer 416. A fully connected layer 413 can be characterized by the fact that a majority, in particular, all edges between nodes 414 of the anterior node layer 414 and the nodes 416 of the posterior node layer are present, and wherein the weight of each of these edges can be adjusted individually.

In this embodiment, the nodes 424 of the anterior node layer 414 of the fully connected layer 415 are displayed both as two-dimensional matrices, and additionally as non-related nodes (indicated as a line of nodes, wherein the number of nodes was reduced for a better presentability). This operation is also denoted as "flattening". In this embodiment, the number of nodes 426 in the posterior node layer 416 of the fully connected layer 415 smaller than the number of nodes 424 in the anterior node layer 414. Alternatively, the number of nodes 426 can be equal or larger.

Furthermore, in this embodiment the Softmax activation function is used within the fully connected layer 415. By applying the Softmax function, the sum the values of all nodes 426 of the output layer 416 is 1, and all values of all nodes 426 of the output layer 416 are real numbers between 0 and 1. In particular, if using the convolutional neural network 400 for categorizing input data, the values of the output layer 416 can be interpreted as the probability of the input data falling into one of the different categories.

In particular, convolutional neural networks 400 can be trained based on the backpropagation algorithm. For preventing overfitting, methods of regularization can be used, e.g., dropout of nodes 420, ..., 424, stochastic pooling, use of artificial data, weight decay based on the L1 or the L2 norm, or max norm constraints.

According to an aspect, the machine learning model may comprise one or more residual networks (ResNet). In particular, a ResNet is an artificial neural network comprising at least one jump or skip connection used to jump over at least one layer of the artificial neural network. In particular, a ResNet may be a convolutional neural network comprising one or more skip connections respectively skipping one or more convolutional layers. According to some examples, the ResNets may be represented as m-layer ResNets, where m is the number of layers in the corresponding architecture and, according to some examples, may take values of 34, 50, 101, or 152. According to some examples, such an m-layer ResNet may respectively comprise (m-2)/2 skip connections.

A skip connection may be seen as a bypass which directly feeds the output of one preceding layer over one or more bypassed layers to a layer succeeding the one or more bypassed layers. Instead of having to directly fit a desired mapping, the bypassed layers would then have to fit a residual mapping "balancing" the directly fed output.

Fitting the residual mapping is computationally easier to optimize than the directed mapping. What is more, this alleviates the problem of vanishing/exploding gradients during optimization upon training the machine learning models: if a bypassed layer runs into such problems, its contribution may be skipped by regularization of the directly fed output. Using ResNets thus brings about the advantage that much deeper networks may be trained.

In particular, a recurrent machine learning model is a machine learning model whose output does not only depend on the input value and the parameters of the machine learning model adapted by the training process, but also on a hidden state vector, wherein the hidden state vector is based on previous inputs used on for the recurrent machine learning model. In particular, the recurrent machine learning model can comprise additional storage states or additional structures that incorporate time delays or comprise feedback loops.

In particular, the underlying structure of a recurrent machine learning model can be a neural network, which can be denoted as recurrent neural network. Such a recurrent neural network can be described as an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network).

In particular, training a recurrent neural network can be based on the BPTT algorithm (acronym for "backpropagation through time"), on the RTRL algorithm (acronym for "real-time recurrent learning") and/or on genetic algorithms.

By using a recurrent machine learning model input data comprising sequences of variable length can be used. In particular, this implies that the method cannot be used only for a fixed number of input datasets (and needs to be trained differently for every other number of input datasets used as input), but can be used for an arbitrary number of input datasets. This implies that the whole set of training data, independent of the number of input datasets contained in different sequences, can be used within the training, and that training data is not reduced to training data corresponding to a certain number of successive input datasets.

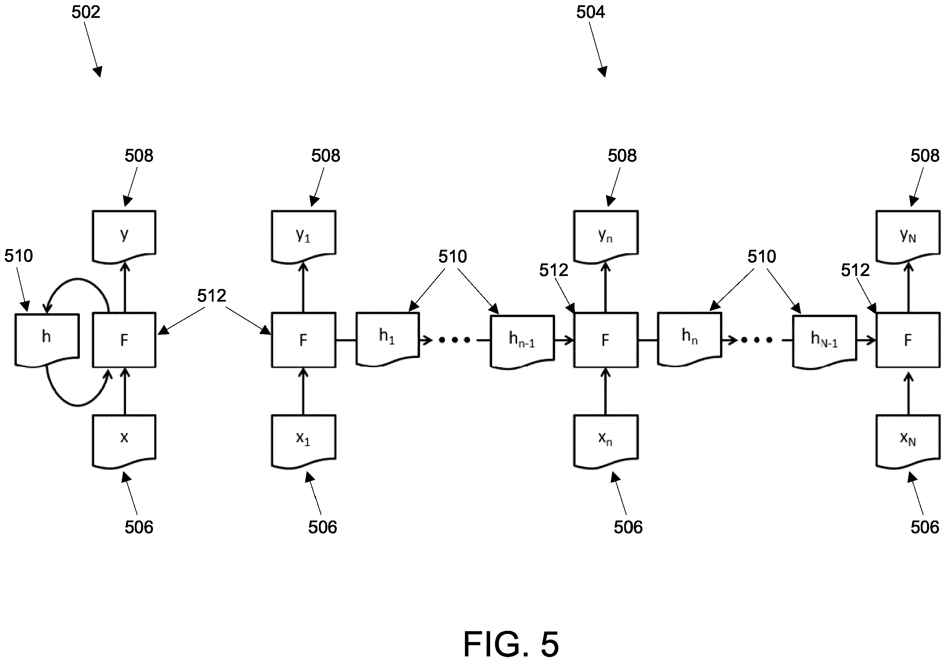

Figure 5 shows the schematic structure of a recurrent machine learning model F, both in a recurrent representation 502 and in an unfolded representation 504, that may be used to implement one or more machine learning models described herein. The recurrent machine learning model takes as input several input datasets $x, x_1, ..., x_N$ 506 and creates a corresponding set of output datasets $y, y_1, ..., y_N$ 508. Furthermore, the output depends on a so-called hidden vector $h, h_1, ..., h_N$ 510, which implicitly comprises information about input datasets previously used as input for the recurrent machine learning model F 512. By using these hidden vectors $h, h_1, ..., h_N$ 510, a sequentiality of the input datasets can be leveraged.

In a single step of the processing, the recurrent machine learning model F 512 takes as input the hidden vector $h_{n-1}$ created within the previous step and an input dataset $x_n$. Within this step, the recurrent machine learning model F generates as output an updated hidden vector $h_n$ and an output dataset $y_n$. In other words, one step of processing calculates $(y_n, h_n) = F(x_n, h_{n-1})$, or by splitting the recurrent machine learning model F 512 into a part F(y) calculating the output data and F(h) calculating the hidden vector, one step of processing calculates $y_n = F^{(y)}(x_n, h_{n-1})$ and $h_n = F^{(h)}(x_n, h_{n-1})$. For the first processing step, $h_0$ can be chosen randomly or filled with all entries being zero. The parameters of the recurrent machine learning model F 512 that were trained based on training datasets before do not change between the different processing steps.

In particular, the output data and the hidden vector of a processing step depend on all the previous input datasets used in the previous steps. $y_n = F^{(y)}(x_n, F^{(h)}(x_{n-1}, h_{n-2}))$ and $h_n = F(h)(x_n, F^{(h)}(x_{n-1}, h_{n-2}))$.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 or 2. Certain steps or functions of the methods and workflows described herein, including one or more of the steps or functions of Figures 1 or 2, may be performed by a server or by another processor in a network-based cloud-computing system. Certain steps or functions of the methods and workflows described herein, including one or more of the steps of Figures 1 or 2, may be performed by a client computer in a network-based cloud computing system. The steps or functions of the methods and workflows described herein, including one or more of the steps of Figures 1 or 2, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 or 2, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 602 that may be used to implement systems, apparatuses, and methods described herein is depicted in Figure 6. Computer 602 includes a processor 604 operatively coupled to a data storage device 612 and a memory 610. Processor 604 controls the overall operation of computer 602 by executing computer program instructions that define such operations. The computer program instructions may be stored in data storage device 612, or other computer readable medium, and loaded into memory 610 when execution of the computer program instructions is desired. Thus, the method and workflow steps or functions of Figures 1 or 2 can be defined by the computer program instructions stored in memory 610 and/or data storage device 612 and controlled by processor 604 executing the computer program instructions. For example, the computer program instructions can be implemented as computer executable code programmed by one skilled in the art to perform the method and workflow steps or functions of Figures 1 or 2. Accordingly, by executing the computer program instructions, the processor 604 executes the method and workflow steps or functions of Figures 1 or 2. Computer 602 may also include one or more network interfaces 606 for communicating with other devices via a network. Computer 602 may also include one or more input/output devices 608 that enable user interaction with computer 602 (e.g., display, keyboard, mouse, speakers, buttons, etc.).

Processor 604 may include both general and special purpose microprocessors, and may be the sole processor or one of multiple processors of computer 602. Processor 604 may include one or more central processing units (CPUs), for example. Processor 604, data storage device 612, and/or memory 610 may include, be supplemented by, or incorporated in, one or more application-specific integrated circuits (ASICs) and/or one or more field programmable gate arrays (FPGAs).

Data storage device 612 and memory 610 each include a tangible non-transitory computer readable storage medium. Data storage device 612, and memory 610, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 608 may include peripherals, such as a printer, scanner, display screen, etc. For example, input/output devices 608 may include a display device such as a cathode ray tube (CRT) or liquid crystal display (LCD) monitor for displaying information to the user, a keyboard, and a pointing device such as a mouse or a trackball by which the user can provide input to computer 602.

An image acquisition device 614 can be connected to the computer 602 to input image data (e.g., medical images) to the computer 602. It is possible to implement the image acquisition device 614 and the computer 602 as one device. It is also possible that the image acquisition device 614 and the computer 602 communicate wirelessly through a network. In a possible embodiment, the computer 602 can be located remotely with respect to the image acquisition device 614.

Any or all of the systems, apparatuses, and methods discussed herein may be implemented using one or more computers such as computer 602.

One skilled in the art will recognize that an implementation of an actual computer or computer system may have other structures and may contain other components as well, and that Figure 6 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope and spirit of the invention. Those skilled in the art could implement various other feature combinations without departing from the scope and spirit of the invention.

The following is a list of non-limiting illustrative embodiments disclosed herein:

Illustrative embodiment 1. A computer-implemented method comprising: receiving first text-based instructions from a user; generating, by an interface AI agent based on the first text-based instructions, 1) second text-based instructions for retrieving clinical information and 2) third text-based instructions for performing a medical analysis task; determining, by a data AI agent based on the second text-based instructions, one or more actions for retrieving the clinical information from one or more clinical information systems; determining, by a task AI agent based on the third text-based instructions, one or more actions for executing on one or more medical applications to perform the medical analysis task; and outputting the clinical information and results of the medical analysis task.

Illustrative embodiment 2. The computer-implemented method of illustrative embodiment 1, wherein determining, by a task AI agent based on the third text-based instructions, one or more actions for executing on one or more medical applications to perform the medical analysis task comprises: determining, by the task AI agent based on the third text-based instructions, additional text-based instructions for retrieving additional clinical information; determining, by the data AI agent based on the additional text-based instructions, one or more actions for retrieving the additional clinical information; and determining, by the task AI agent further based on the additional clinical information, the one or more actions for executing on the one or more medical applications.

Illustrative embodiment 3. The computer-implemented method of any of illustrative embodiments 1-2, wherein generating, by an interface AI agent based on the first text-based instructions, 1) second text-based instructions for retrieving clinical information and 2) third text-based instructions for performing a medical analysis task comprises: generating, by the interface AI agent based on the first text-based instructions, text-based follow-up instructions requesting additional information from the user; and generating, by the interface AI agent further based on a response to the text-based follow-up instructions received from the user, 1) the second text-based instructions and 2) the third text-based instructions.

Illustrative embodiment 4. The computer-implemented method of any of illustrative embodiments 1-3, wherein the one or more actions for retrieving the clinical information comprises at least one of: searching, classifying, parsing, or interpreting clinical data stored in the one or more clinical information systems.

Illustrative embodiment 5. The computer-implemented method of any of illustrative embodiments 1-4, wherein the one or more actions for executing on the one or more medical applications comprise at least one of: one or more medical image analysis tasks performed on one or more medical images, functions to derive findings from the one or more medical images, functions to apply geometric transformations on the one or more medical images, functions to retrieve geometric information from the one or more medical images, or outputting text-based instructions to a machine learning based model.

Illustrative embodiment 6. The computer-implemented method of any of illustrative embodiments 1-5, wherein at least one of the one or more actions for retrieving the clinical information or the one or more actions for executing on the one or more medical applications comprise one or more APIs (application programming interfaces).

Illustrative embodiment 7. The computer-implemented method of any of illustrative embodiments 1-6, wherein receiving first text-based instructions from a user comprises: receiving spoken instructions from the user; and converting the spoken instructions to the first text-based instructions.

Illustrative embodiment 8. The computer-implemented method of any of illustrative embodiments 1-7, wherein the interface AI agent, the data AI agent, and the task AI agent each comprise a machine learning based text encoder network and a policy module.

Illustrative embodiment 9. The computer-implemented method of any one of illustrative embodiments 1-8, wherein the first text-based instructions are at least one of predefined based on a specific medical application or automatically generated based on contextual information of the user or patient.

Illustrative embodiment 10. An apparatus comprising: means for receiving first text-based instructions from a user; means for generating, by an interface AI agent based on the first text-based instructions, 1) second text-based instructions for retrieving clinical information and 2) third text-based instructions for performing a medical analysis task; means for determining, by a data AI agent based on the second text-based instructions, one or more actions for retrieving the clinical information from one or more clinical information systems; means for determining, by a task AI agent based on the third text-based instructions, one or more actions for executing on one or more medical applications to perform the medical analysis task; and means for outputting the clinical information and results of the medical analysis task.

Illustrative embodiment 11. The apparatus of illustrative embodiment 10, wherein the means for determining, by a task AI agent based on the third text-based instructions, one or more actions for executing on one or more medical applications to perform the medical analysis task comprises: means for determining, by the task AI agent based on the third text-based instructions, additional text-based instructions for retrieving additional clinical information; means for determining, by the data AI agent based on the additional text-based instructions, one or more actions for retrieving the additional clinical information; and means for determining, by the task AI agent further based on the additional clinical information, the one or more actions for executing on the one or more medical applications.

Illustrative embodiment 12. The apparatus of any of illustrative embodiments 10-11, wherein the means for generating, by an interface AI agent based on the first text-based instructions, 1) second text-based instructions for retrieving clinical information and 2) third text-based instructions for performing a medical analysis task comprises: means for generating, by the interface AI agent based on the first text-based instructions, text-based follow-up instructions requesting additional information from the user; and means for generating, by the interface AI agent further based on a response to the text-based follow-up instructions received from the user, 1) the second text-based instructions and 2) the third text-based instructions.

Illustrative embodiment 13. The apparatus of any of illustrative embodiments 10-12, wherein the one or more actions for retrieving the clinical information comprises at least one of: searching, classifying, parsing, or interpreting clinical data stored in the one or more clinical information systems.

Illustrative embodiment 14. The apparatus of any of illustrative embodiments 10-13, wherein the one or more actions for executing on the one or more medical applications comprise at least one of: one or more medical image analysis tasks performed on one or more medical images, functions to derive findings from the one or more medical images, functions to apply geometric transformations on the one or more medical images, functions to retrieve geometric information from the one or more medical images, or outputting text-based instructions to a machine learning based model.

Illustrative embodiment 15. A non-transitory computer-readable storage medium comprising instructions which, when executed by a computer, cause the computer to carry out operations comprising: receiving first text-based instructions from a user; generating, by an interface AI agent based on the first text-based instructions, 1) second text-based instructions for retrieving clinical information and 2) third text-based instructions for performing a medical analysis task; determining, by a data AI agent based on the second text-based instructions, one or more actions for retrieving the clinical information from one or more clinical information systems; determining, by a task AI agent based on the third text-based instructions, one or more actions for executing on one or more medical applications to perform the medical analysis task; and outputting the clinical information and results of the medical analysis task.

Illustrative embodiment 16. The non-transitory computer-readable storage medium of illustrative embodiment 15, wherein determining, by a task AI agent based on the third text-based instructions, one or more actions for executing on one or more medical applications to perform the medical analysis task comprises: determining, by the task AI agent based on the third text-based instructions, additional text-based instructions for retrieving additional clinical information; determining, by the data AI agent based on the additional text-based instructions, one or more actions for retrieving the additional clinical information; and determining, by the task AI agent further based on the additional clinical information, the one or more actions for executing on the one or more medical applications.

Illustrative embodiment 17. The non-transitory computer-readable storage medium of any of illustrative embodiments 15-16, wherein at least one of the one or more actions for retrieving the clinical information or the one or more actions for executing on the one or more medical applications comprise one or more APIs (application programming interfaces).

Illustrative embodiment 18. The non-transitory computer-readable storage medium of any of illustrative embodiments 15-17, wherein receiving first text-based instructions from a user comprises: receiving spoken instructions from the user; and converting the spoken instructions to the first text-based instructions.

Illustrative embodiment 19. The non-transitory computer-readable storage medium of any of illustrative embodiments 15-18, wherein the interface AI agent, the data AI agent, and the task AI agent each comprise a machine learning based text encoder network and a policy module.

Illustrative embodiment 20. The non-transitory computer-readable storage medium of any of illustrative embodiments 15-19, wherein the first text-based instructions are at least one of predefined based on a specific medical application or automatically generated based on contextual information of the user or patient.

**Claims**

1. A computer-implemented method comprising:

   receiving (102) first text-based instructions (204) from a user (202);
   generating (104), by an interface AI agent (208) based on the first text-based instructions, 1) second text-based instructions (210) for retrieving clinical information and 2) third text-based instructions (212) for performing a medical analysis task;
   determining (106), by a data AI agent (214) based on the second text-based instructions, one or more actions (216) for retrieving the clinical information from one or more clinical information systems (218);

determining (108), by a task AI agent (220) based on the third text-based instructions, one or more actions (222) for executing on one or more medical applications (226) to perform the medical analysis task; and

outputting (110) the clinical information and results of the medical analysis task.

2. The computer-implemented method of claim 1, wherein determining, by a task AI agent based on the third text-based instructions, one or more actions for executing on one or more medical applications to perform the medical analysis task comprises:

determining, by the task AI agent based on the third text-based instructions, additional text-based instructions (224) for retrieving additional clinical information;

determining, by the data AI agent based on the additional text-based instructions, one or more actions for retrieving the additional clinical information; and

determining, by the task AI agent further based on the additional clinical information, the one or more actions for executing on the one or more medical applications.

3. The computer-implemented method of claim 1, wherein generating, by an interface AI agent based on the first text-based instructions, 1) second text-based instructions for retrieving clinical information and 2) third text-based instructions for performing a medical analysis task comprises:

generating, by the interface AI agent based on the first text-based instructions, text-based follow-up instructions (206) requesting additional information from the user; and

generating, by the interface AI agent further based on a response to the text-based follow-up instructions received from the user, 1) the second text-based instructions and 2) the third text-based instructions.

4. The computer-implemented method of claim 1, wherein the one or more actions for retrieving the clinical information comprises at least one of: searching, classifying, parsing, or interpreting clinical data stored in the one or more clinical information systems.

5. The computer-implemented method of claim 1, wherein the one or more actions for executing on the one or more medical applications comprise at least one of: one or more medical image analysis tasks performed on one or more medical images, functions to derive findings from the one or more medical images, functions to apply geometric transformations on the one or more medical images, functions to retrieve geometric information from the one or more medical images, or outputting text-based instructions to a machine learning based model.

6. The computer-implemented method of claim 1, wherein at least one of the one or more actions for retrieving the clinical information or the one or more actions for executing on the one or more medical applications comprise one or more APIs (application programming interfaces).

7. The computer-implemented method of claim 1, wherein receiving first text-based instructions from a user comprises:

receiving spoken instructions from the user; and

converting the spoken instructions to the first text-based instructions.

8. The computer-implemented method of claim 1, wherein the interface AI agent, the data AI agent, and the task AI agent each comprise a machine learning based text encoder network and a policy module.

9. The computer-implemented method of claim 1, wherein the first text-based instructions are at least one of predefined based on a specific medical application or automatically generated based on contextual information of the user or patient.

10. An apparatus comprising:

means for receiving (102) first text-based instructions (204) from a user (202);

means for generating (104), by an interface AI agent (208) based on the first text-based instructions, 1) second text-based instructions (210) for retrieving clinical information and 2) third text-based instructions (212) for performing a medical analysis task;

means for determining (106), by a data AI agent (214) based on the second text-based instructions, one or more actions (216) for retrieving the clinical information from one or more clinical information systems (218);

means for determining (108), by a task AI agent (220) based on the third text-based instructions, one or more actions (222) for executing on one or more medical applications (226) to perform the medical analysis task; and
means for outputting (110) the clinical information and results of the medical analysis task.

11. The apparatus of claim 10, wherein the means for determining, by a task AI agent based on the third text-based instructions, one or more actions for executing on one or more medical applications to perform the medical analysis task comprises:

means for determining, by the task AI agent based on the third text-based instructions, additional text-based instructions (224) for retrieving additional clinical information;
means for determining, by the data AI agent based on the additional text-based instructions, one or more actions for retrieving the additional clinical information; and
means for determining, by the task AI agent further based on the additional clinical information, the one or more actions for executing on the one or more medical applications.

12. The apparatus of claim 10, wherein the means for generating, by an interface AI agent based on the first text-based instructions, 1) second text-based instructions for retrieving clinical information and 2) third text-based instructions for performing a medical analysis task comprises:

means for generating, by the interface AI agent based on the first text-based instructions, text-based follow-up instructions (206) requesting additional information from the user; and
means for generating, by the interface AI agent further based on a response to the text-based follow-up instructions received from the user, 1) the second text-based instructions and 2) the third text-based instructions.

13. The apparatus of claim 10, wherein the one or more actions for retrieving the clinical information comprises at least one of: searching, classifying, parsing, or interpreting clinical data stored in the one or more clinical information systems.

14. The apparatus of claim 10, wherein the one or more actions for executing on the one or more medical applications comprise at least one of: one or more medical image analysis tasks performed on one or more medical images, functions to derive findings from the one or more medical images, functions to apply geometric transformations on the one or more medical images, functions to retrieve geometric information from the one or more medical images, or outputting text-based instructions to a machine learning based model.

15. A non-transitory computer-readable storage medium comprising instructions which, when executed by a computer, cause the computer to carry out operations comprising:

receiving (102) first text-based instructions (204) from a user (202);
generating (104), by an interface AI agent (208) based on the first text-based instructions, 1) second text-based instructions (210) for retrieving clinical information and 2) third text-based instructions (212) for performing a medical analysis task;
determining (106), by a data AI agent (214) based on the second text-based instructions, one or more actions (216) for retrieving the clinical information from one or more clinical information systems (218);
determining (108), by a task AI agent (220) based on the third text-based instructions, one or more actions (222) for executing on one or more medical applications (226) to perform the medical analysis task; and
outputting (110) the clinical information and results of the medical analysis task.

16. The non-transitory computer-readable storage medium of claim 15, wherein determining, by a task AI agent based on the third text-based instructions, one or more actions for executing on one or more medical applications to perform the medical analysis task comprises:

determining, by the task AI agent based on the third text-based instructions, additional text-based instructions (224) for retrieving additional clinical information;
determining, by the data AI agent based on the additional text-based instructions, one or more actions for retrieving the additional clinical information; and
determining, by the task AI agent further based on the additional clinical information, the one or more actions for executing on the one or more medical applications.

17. The non-transitory computer-readable storage medium of claim 15, wherein at least one of the one or more actions for retrieving the clinical information or the one or more actions for executing on the one or more medical applications comprise one or more APIs (application programming interfaces).

18. The non-transitory computer-readable storage medium of claim 15, wherein receiving first text-based instructions from a user comprises:

receiving spoken instructions from the user; and
converting the spoken instructions to the first text-based instructions.

19. The non-transitory computer-readable storage medium of claim 15, wherein the interface AI agent, the data AI agent, and the task AI agent each comprise a machine learning based text encoder network and a policy module.

20. The non-transitory computer-readable storage medium of claim 15, wherein the first text-based instructions are at least one of predefined based on a specific medical application or automatically generated based on contextual information of the user or patient.

# FIG. 1

100

```
┌─────────────────────────────────────────────────────────┐
│                                                           │
│         Receive first text-based instructions from a user │
│                            102                            │
│                                                           │
└─────────────────────────────────────────────────────────┘
                              │
                              ▼
┌─────────────────────────────────────────────────────────┐
│                                                           │
│     Generate, by an interface AI agent based on the       │
│     first text-based instructions, 1) second text-based   │
│     instructions for retrieving clinical information      │
│     and 2) third text-based instructions for performing   │
│     a medical analysis task                               │
│                            104                            │
│                                                           │
└─────────────────────────────────────────────────────────┘
                              │
                              ▼
┌─────────────────────────────────────────────────────────┐
│                                                           │
│      Determine, by a data AI agent based on the second    │
│      text-based instructions, one or more actions for     │
│      retrieving the clinical information from one or       │
│      more clinical information systems                     │
│                            106                            │
│                                                           │
└─────────────────────────────────────────────────────────┘
                              │
                              ▼
┌─────────────────────────────────────────────────────────┐
│                                                           │
│   Determine, by a task AI agent based on the third        │
│   text-based instructions, one or more actions for        │
│   executing on one or more medical applications to        │
│   perform the medical analysis task                       │
│                            108                            │
│                                                           │
└─────────────────────────────────────────────────────────┘
                              │
                              ▼
┌─────────────────────────────────────────────────────────┐
│                                                           │
│  Output the clinical information and/or results of the    │
│  medical analysis task                                    │
│                            110                            │
│                                                           │
└─────────────────────────────────────────────────────────┘
```

FIG. 2

FIG. 3

FIG. 4

FIG. 5

EP 4 718 467 A1

# FIG. 6

Europäisches
Patentamt

European
Patent Office

Office européen
des brevets

## EUROPEAN SEARCH REPORT

Application Number

EP 24 46 5578

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/156921 A1 (KOHLI NAMITA [US] ET AL) 23 May 2019 (2019-05-23)<br>* figures 8-11,14 *<br>* paragraphs [0031] - [0033] *<br>* paragraphs [0140] - [0143] *<br>* paragraphs [0100], [0101] *<br>* paragraph [0067] *<br>* paragraph [0159] *<br>----- | 1-20 | INV.<br>G16H30/40<br>G16H50/20 |
| A | US 2023/274420 A1 (SEAH JARREL [AU] ET AL) 31 August 2023 (2023-08-31)<br>* paragraphs [0016], [0017] *<br>* paragraph [0039] - paragraph [0044] *<br>* paragraph [0096] *<br>----- | 1-20 | |
| A | US 2023/076903 A1 (CONJETI SAILESH [DE]) 9 March 2023 (2023-03-09)<br>* paragraph [0043] - paragraph [0053]; figure 1 *<br>----- | 1-20 | |
| | | | TECHNICAL FIELDS SEARCHED (IPC)<br><br>G16H |

The present search report has been drawn up for all claims

| Place of search | Date of completion of the search | Examiner |
|---|---|---|
| Munich | 11 March 2025 | Megalou-Nash, M |

EPO FORM 1503 03.82 (P04C01)

## ANNEX TO THE EUROPEAN SEARCH REPORT
## ON EUROPEAN PATENT APPLICATION NO.

EP 24 46 5578

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2025

| Patent document cited in search report | Publication date | Patent family member(s) | | Publication date |
|---|---|---|---|---|
| US 2019156921 A1 | 23-05-2019 | US | 2019156921 A1 | 23-05-2019 |
| | | WO | 2019104093 A1 | 31-05-2019 |
| US 2023274420 A1 | 31-08-2023 | AU | 2021306421 A1 | 23-02-2023 |
| | | EP | 4176449 A1 | 10-05-2023 |
| | | US | 2023274420 A1 | 31-08-2023 |
| | | WO | 2022006621 A1 | 13-01-2022 |
| US 2023076903 A1 | 09-03-2023 | EP | 4141878 A1 | 01-03-2023 |
| | | US | 2023076903 A1 | 09-03-2023 |

EPO FORM P0459

For more details about this annex : see Official Journal of the European Patent Office, No. 12/82